# EUROPEAN PATENT SPECIFICATION

(11) **EP 2 797 584 B1**
(45) Date of publication and mention of the grant of the patent: **16.12.2015**
(21) Application number: 12834605.3
(22) Date of filing: 25.12.2012
(51) Int. Cl.: A61K 9/00, A61K 31/00, A61K 47/00

(54) **COMBINATIONS OF DIACEREIN AND NON-STEROIDAL INFLAMMATION DRUGS**
KOMBINATIONEN AUS DIACEREIN UND NICHTSTEROIDEN ENTZÜNDUNGSHEMMENDEN MITTELN
COMBINAISONS DE DIACÉRÉINE ET D'ANTI-INFLAMMATOIRES NON STÉROÏDIENS

(30) Priority: 27.12.2011 TR 201113006; 01.08.2012 TR 201208987; 14.12.2012 TR 201214658
(43) Date of publication of application: 05.11.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); PEHLIVAN AKALIN, Nur, 34460 Istanbul (TR); ZENGINER, Sibel, 34460 Istanbul (TR); MUTLU, Onur, 34460 Istanbul (TR)
(74) Representative: Sevinç, Erkan
(86) International application number: PCT/TR2012/000247
(87) International publication number: WO 2013/100882

(56) References cited:
- GB-A- 2 432 526
- US-A- 4 996 209
- US-A1- 2004 039 366
- ÁLVAREZ-SORIA M A ET AL: "Diacerein has a weak effect on the catabolic pathway of human osteoarthritis synovial fibroblast", RHEUMATOLOGY, vol. 47, no. 5, 27 March 2008 (2008-03-27) , pages 627-633, XP009173169, ISSN: 1462-0324 [retrieved on 2008-03-27]

## Description

### Field of Invention

The present invention relates to novel pharmaceutical combinations with synergistic action of diacerein or a pharmaceutically acceptable salt of diacerein and non-steroidal inflammatory drugs, having analgesic, anti-inflammatory, antipyretic, and osteoarthritis-treating activities.

### Background of Invention

Diacerein, having the chemical structure given below with Formula 1, is an interleukin-1 inhibitor having an anthraquinone structure and is used for treating osteoarthritis.

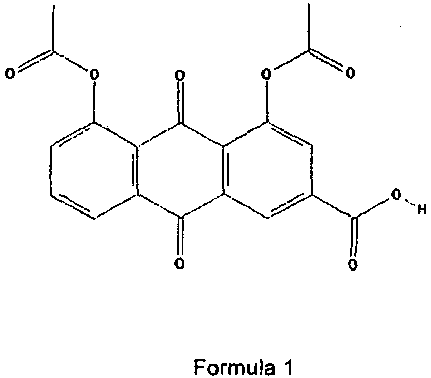

Studies conducted on humans and animals have shown that diacerein alleviates the symptoms of osteoarthritis. It was further reported that diacerein reduces the osteoarthritis symptoms, particularly the pain, in a statistically significant manner and provides positive structural effects, when compared to placebo. The symptomatic effect of diacerein starts 30 - 45 days after the initiation of treatment. The structure modifying effect of diacerein was proposed based on the findings of the ECHODIAH study, a randomized double-blind study. Diacerein is accepted as a slow-acting drug against osteoarthritis.

Diacerein and more specifically rhein, which is an active metabolite thereof, is an IL-1 inhibitor. Rhein is an anthraquinone present in plants like the Cassia species and has moderate analgesic, antipyretic, and anti-inflammatory effects. It has a weak laxative effect. Diacerein inhibits human monocytes in vitro. The increase in collagenase formation mediated by IL-1 in chondrocytes is actively inhibited by diacerein. Diacerein is administered orally. Following oral administration, diacerein undergoes deacetylation before it enters the systemic circulation, and is absorbed, metabolized and is excreted in the form of rhein and the conjugates thereof.

Various patent applications have been filed which relate to diacerein.

The patent documents US 4,244,968 and EP0636602 mention on the use of diacerein in the treatment of arthritis.

The document EP1248608 makes mention on the use of diacerein in the treatment of psoriazis.

Non-steroidal inflammatory drugs (NSAID) have antipyretics, analgesic, anti-inflammatory activities. The main NSAID'S are flurbiprofen, loxoprofen, zaltoprofen, ibuprofen, naproxen, ketoprofen, dexketoprofen, fenoprofen, benoxaprofen, suprofen, ibuproxam, alminoprofen, dexibuprofen, indoprofen.

Flurbiprofen is a propionic acid derivative, also known as NSAID (non-steroidal anti-inflammatory drug), having analgesic and anti-inflammatory activities. Its chemical structure is illustrated with Formula 2 given below.

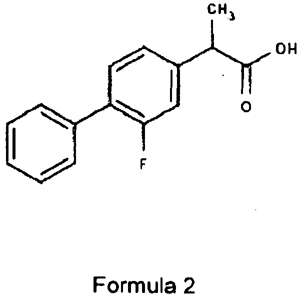

Flurbiprofen is used for alleviating pain in the muscle-skeleton system and joint disorders such as ankylosing spondylitis, osteoarthritis, and rheumatoid arthritis, in soft tissue injuries such as sprains and strains, in postoperative cases, and in painful and severe menstruation and migraine. Flurbiprofen is further used as a lozenge in the symptomatic amelioration of sore throats.

The document US3755427 describes the flurbiprofen molecule and the anti-inflammatory, analgesic, antipyretic and anti-toxic effects of flurbiprofen.

The document US4014993 discloses the use of flurbiprofen in platelet aggregation.

The document EP137668 discloses the use of flurbiprofen in the treatment of alveolar bone resorption.

The document EP1655026 discloses a combination of diacerein and meloxicam.

No orally-administrable pharmaceutical composition has been produced until today, which contains a combination of flurbiprofen and diacerein. Even if some medicaments comprising either of these active agents have been administered concomitantly in practice, this fact requires the patients to carry more than one drug and causes application-related difficulties. Additionally, administering and formulating a combination, in place of the individual use of each active agent, may provide improved treatment features.

In result, based on said drawbacks, a novelty is required in the art of pharmaceutical compositions having therapeutic effects against pain, inflammation, fewer, and osteoarthritis.

### Object and Brief Description of Invention

The present invention provides a composition with synergistic action of diacerein and least one agent selected from the group of non-steroidal anti-inflammatory agents consisting of flurbiprofen, loxoprofen, zaltoprofen, ibuprofen, naproxen, ketoprofen, dexketoprofen, fenoprofen, benoxaprofen, suprofen, ibuproxam, alminoprofen, dexibuprofen, indoprofen, eliminating all aforesaid problems and brining additional advantages to the relevant prior art.

Accordingly, the main object of the present invention is to obtain a combination composition.

Another object of the present invention is to obtain a composition with synergistic action having analgesic activity.

Another object of the present invention is to obtain a composition with synergistic action having artritis-treating activity.

Another object of the present invention is to obtain a composition with synergistic action having inflammation-treating activity.

Another object of the present invention is to obtain a composition with synergistic action having antipyretic activity.

Another object of the present invention is to obtain a combination composition with synergistic action having therapeutic effects against inflammation, pain, fewer, and osteoarthritis.

A further object of the present invention is to obtain a combination composition having content uniformity and a desired level of compatibility.

In order to achieve the objects described above and to emerge in the following detailed description, a composition is developed for use in the treatment of pain, inflammation, fewer, and osteoarthritis.

In a preferred embodiment according to the present invention, said novelty comprises diacerein or a pharmaceutically acceptable salt of diacerein and at least one agent selected from the group of non-steroidal anti-inflammatory agents consisting of flurbiprofen, loxoprofen, zaltoprofen, ibuprofen, naproxen, ketoprofen, dexketoprofen, fenoprofen, benoxaprofen, suprofen, ibuproxam, alminoprofen, dexibuprofen, indoprofen.

In another preferred embodiment according to the present invention, said non-steroidal anti-inflammatory active agent is flurbiprofen.

In another preferred embodiment according to the present invention, said non-steroidal anti-inflammatory active agent is loxoprofen.

In another preferred embodiment according to the present invention, said non-steroidal anti-inflammatory active agent is zaltoprofen.

In another preferred embodiment according to the present invention, said non-steroidal anti-inflammatory active agent is ibuprofen.

In another preferred embodiment according to the present invention, said non-steroidal anti-inflammatory active agent is ketoprofen or dexketoprofen.

In another preferred embodiment according to the present invention, said non-steroidal anti-inflammatory active agent is naproxen.

In a preferred embodiment according to the present invention, the amount of diacerein is 50 - 300 mg/day.

In a preferred embodiment according to the present invention, the amount of flurbiprofen is 50 - 500 mg/day.

In a preferred embodiment according to the present invention, the amount of loxoprofen is 50 - 400 mg/day.

In a preferred embodiment according to the present invention, the amount of zaltoprofen is 50 - 500 mg/day.

In a preferred embodiment according to the present invention, the amount of ibuprofen is 50 - 1000 mg/day.

In a preferred embodiment according to the present invention, the amount of ketoprofen or dexketoprofen is 25 - 400 mg/day.

In a preferred embodiment according to the present invention, the amount of naproxen is 200 - 800 mg/day.

In another preferred embodiment according to the present invention, said proportion range of diacerein to the non-steroidal anti-inflammatory agents is 0.01 - 4.

Another preferred embodiment according to the present invention comprises one selected from nimesulide, s-etodolac, glucosamine, methylsulfonylmethane and chondroitin.

In a preferred embodiment according to the present invention, the composition is a fixed dose or a kit.

In a preferred embodiment according to the present invention, the composition is in the form of an oral solid or liquid or a topical liquid or gel.

In a preferred embodiment according to the present invention, said oral solid form is a tablet or capsule.

Another preferred embodiment of the present invention further comprises at least one or more excipient(s).

In a preferred embodiment according to the present invention, said excipient(s) comprise(s) one or more selected from the group consisting of diluents, binders, disintegrants, glidants, lubricants, solvents, pH regulators, gelling agents, and plasticizers.

Another embodiment according to the present invention is the use of each active agent in said composition simultaneously or in sequence in any order, separately or in a fixed combination.

A further embodiment according to the present invention is the use of said composition comprising diacerein or a pharmaceutically acceptable salt of diacerein and at least one agent selected from the group of non-steroidal anti-inflammatory agents consisting of flurbiprofen, ibuprofen, naproxen, ketoprofen, dexketoprofen, fenoprofen, benoxaprofen, suprofen, ibuproxam, alminoprofen, dexibuprofen, indoprofen for the treatment of pain, inflammation, and osteoarthritis.

### Detailed Description of Invention

### Example 1: Tablet or capsule formulation

### a) Hot Melt Granulation Method

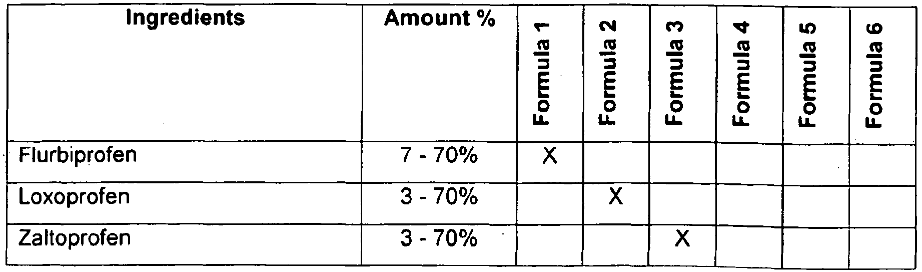

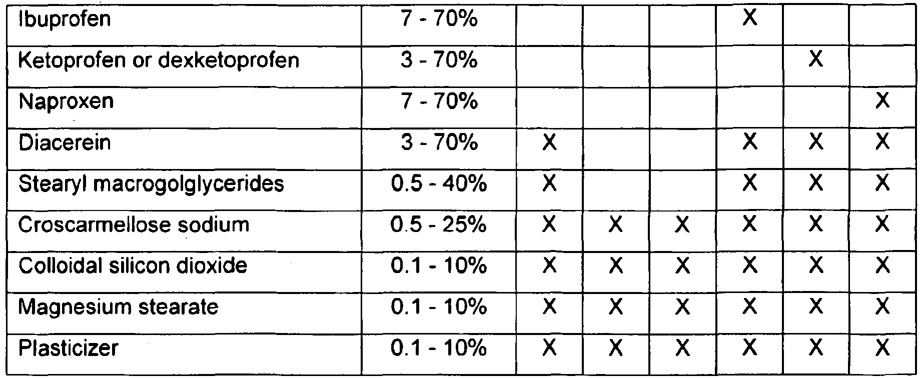

Each column defines the content of a formulation, wherein the active agents and excipients included to the formulation are used for granulation in the processes according to the prior art. The X sign corresponding to each column and line indicates the excipients contained in the formula of the respective column.

The formulation is produced as follows: at least one of the non-steroidal inflammation drugs, diacerein, plasticizer, and stearyl macrogolglycerides are mixed together and melted and then passed through an extruder or a sieve. Into the granules obtained above, first croscarmellose sodium and colloidal silicon dioxide, and then magnesium stearate are added and the resulting mixture is mixed. A compression step is performed on this powder mixture in a tablet machine, or this powder mixture is filled into capsules. The tablets are coated preferably with a humidity-barrier coating material, such as Opadry amb/Kollicoat IR.

### b) Wet Granulation

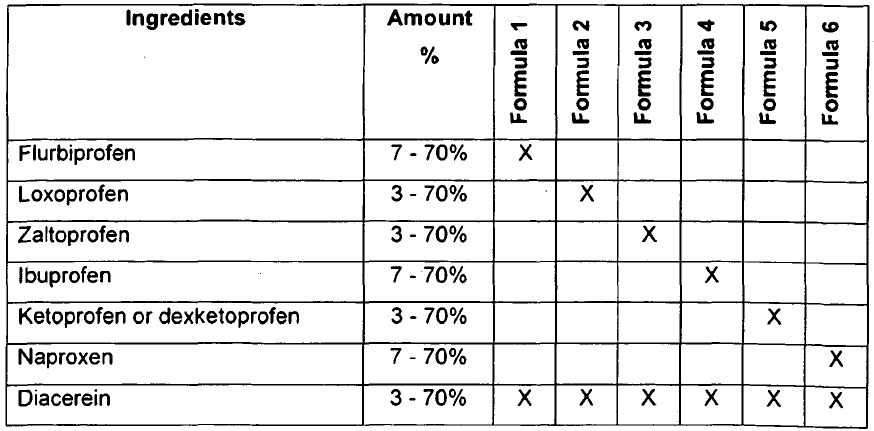

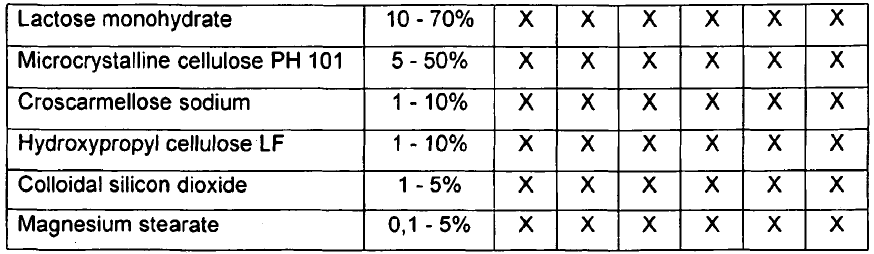

Each column defines the content of a formulation, wherein the active agents and excipients included to the formulation are used for granulation in the processes according to the prior art. The X sign corresponding to each column and line indicates the excipients contained in the formula of the respective column.

At least one of the non-steroidal inflammation drugs, microcrystalline cellulose (PH101), lactose monohydrate, and some amount of hydroxypropyl cellulose are loaded to a fluidized bed dryer. An aqueous solution is prepared with the remaining amount of hydroxypropyl cellulose. It is granulated in the fluidized bed dryer, dried and ground and taken to a container. Croscarmellose sodium and colloidal silicon dioxide are added to the same container. The powder mixture in this container is mixed for ca. 10 minutes. Then, magnesium stearate is added to the resulting mixture and mixed for ca. 3 more minutes. A compression step is performed on the resulting mixture in a tablet machine, or this powder mixture is filled into capsules.

### c) Direct compression

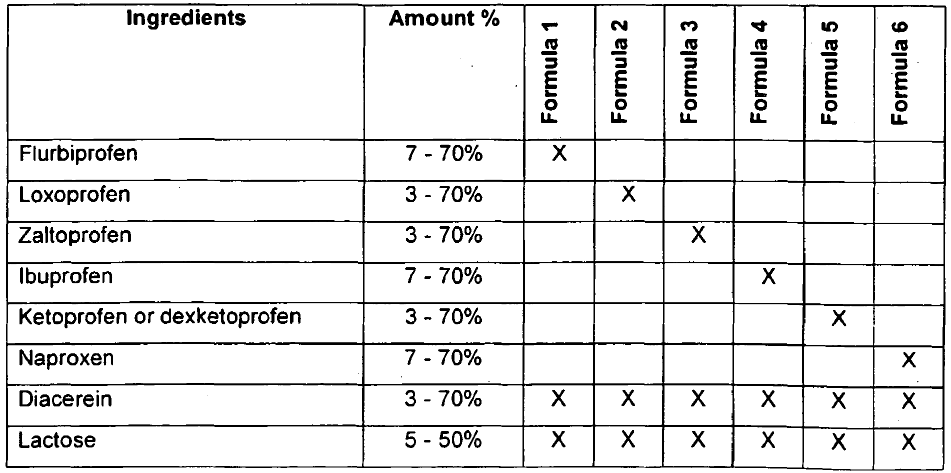

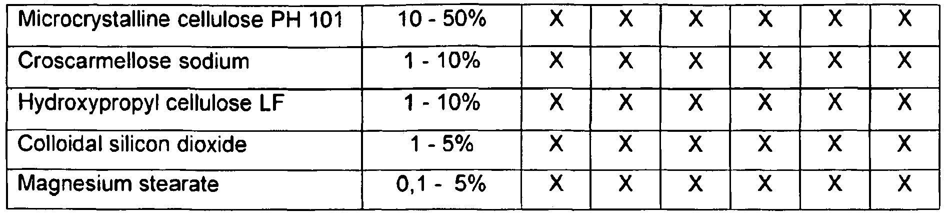

Each column defines the content of a formulation, wherein the active agents and excipients included to the formulation are used for granulation in the processes according to the prior art. The X sign corresponding to each column and line indicates the excipients contained in the formula of the respective column.

The active agents and excipients contained in the formulation are used for granulation in the processes according to the prior art.

### Example 2: Granule Formulation for Oral Suspension

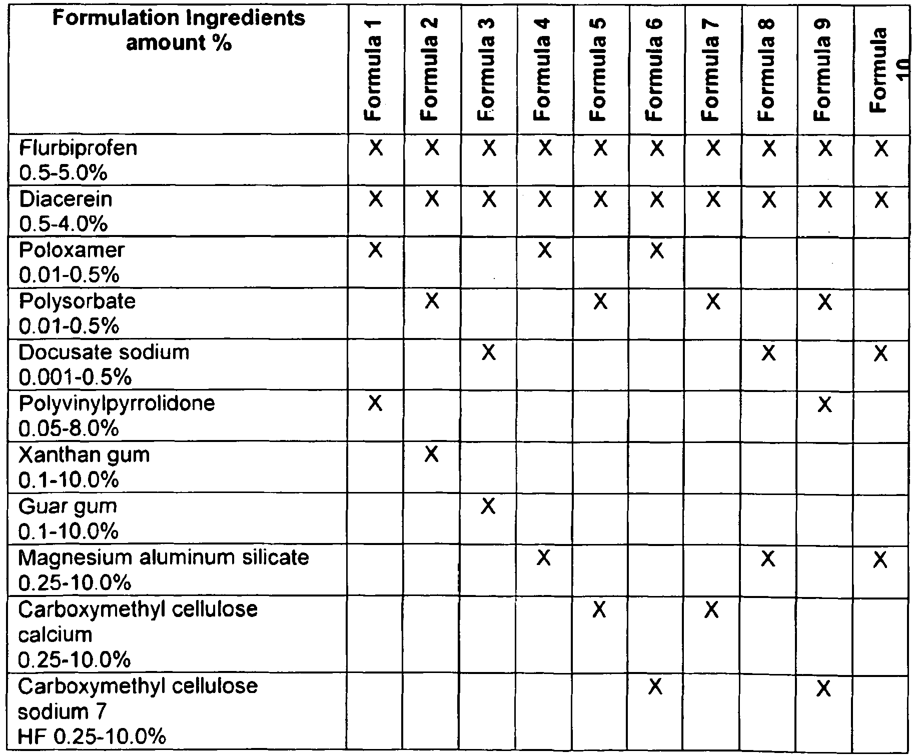

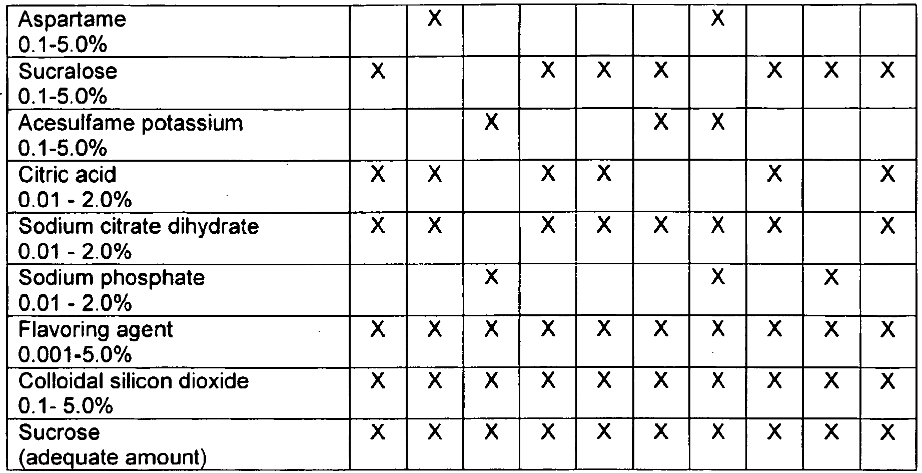

Each column defines the content of a formulation, wherein the active agents and excipients included to the formulation are used for granulation in the processes according to the prior art. The X sign corresponding to each column and line indicates the excipients contained in the formula of the respective column.

Among the excipients indicated for the formulation, suspension agents (polyvinylpyrrolidone, xanthan gum, guar gum, magnesium aluminum silicate, carboxymethyl cellulose calcium and carboxymethyl cellulose sodium 7 HF), surface active agents (Poloxamer, Tween 80, docusate sodium) and buffering agents (citric acid, sodium citrate dihydrate and sodium phosphate) directly influence the product stability.

### Example 3: Topical formulation

| **FLURBIPROFEN DIACEREIN TOPICAL GEL amount %** | |
|---|---|
| Flurbiprofen | 0.5 - 5.0% |
| Diacerein | 0.5 - 4.0% |
| Ethyl alcohol (96%) | 2 - 25% |
| Carbomer 940 | 0.25 - 5% |
| Polyethylene glycol 400 | 2 - 30% |
| Polysorbate 80 | 0.1 - 5% |
| Triethanolamine | 0.1 - 5% |
| Glycerin | 2 - 30% |
| Dimethylsulfoxide | 2 - 20% |
| Methylparaben | 0.01 - 32% |
| Propylparaben | 0.01 - 2% |
| Lavender essence | 0.02 - 1% |
| Purified water | adequate amount |

The active agents and excipients contained in the formulation are used for producing gel in the processes according to the prior art.

| **FLURBIPROFEN DIACEREIN TOPICAL GEL amount %** | |
|---|---|
| Flurbiprofen | 0.5 - 5.0% |
| Diacerein | 0.5 - 4.0% |
| Menthol | 0.25 - 15% |
| Dimethyl sulfoxide | 2 - 20% |
| Hydroxypropyl cellulose H | 0.25 - 10% |
| Polyethylene glycol 400 | 2 - 30% |
| Polysorbate 80 | 0.1 - 5% |
| Glycerin | 2 - 30% |
| Sorbitol 70 | 2 - 30% |
| Triethanolamine | 0.1 - 5% |
| Ethyl alcohol | adequate amount |

The active agents and excipients contained in the formulation are used for producing gel in the processes according to the prior art.

| **FLURBIPROFEN DIACEREIN TOPICAL CREAM** | |
|---|---|
| **amount %** | |
| Flurbiprofen | 0.5 - 5.0% |
| Diacerein | 0.5 - 4.0% |
| Liquid paraffin | 2 - 30% |
| Cetostearyl alcohol | 1 - 15% |
| Methylparaben | 0.01 - 2% |
| Propylparaben | 0.01 - 2% |
| Glycerin | 2 - 30% |
| Propylene glycol | 2 - 50% |
| White beeswax | 0.5 - 10% |
| Sodium metabisulfite | 0.1 - 8% |
| Benzyl alcohol | 0.1 - 5% |
| Lavender oil | 0.01 - 1% |
| water | adequate amount |

The active agents and excipients contained in the formulation are used for cream production in the processes according to the prior art.

With the present invention, a combination composition with synergistic action is surprisingly obtained, which has therapeutic effects against pain, inflammation, fewer, and osteoarthritis. Under normal conditions, the action of diacerein against osteoarthritis is slow. The action time, however, is reduced with the present invention.

Drugs of different action mechanisms can be combined. It is not possible, however, to state that a combination of drugs having different action mechanisms, but showing actions on similar targets, will have absolutely positive effects.

The term synergistic means that when drugs are administered together, a combined action is obtained which is larger than the sum of individual actions of the respective drugs when they are used separately. On the other hand, using a lower dose of each drug to be combined according to the present invention will reduce the total dosage. Put differently, the dosages have not to be relatively less in all cases, but the drugs can be dosed less frequently or this may be beneficial in reducing the recurrence rate of side effects. These are advantageous in terms of patients to be treated.

The preferred dosages of active agents included to the pharmaceutical combination according to the present invention are therapeutically active dosages, and particularly correspond to the dosage of those which are commercially available. Therapeutically active amount not only includes therapeutic doses, but also preventive/prophylactic doses.

The present invention further relates to a commercial package, comprising a combination of the present invention together with instructions for the use thereof in a simultaneous, separate, or sequential use.

These pharmaceutical preparations are for oral, topical, parenteral or rectal administrations and comprise the pharmacologically active agent either alone, or together with pharmaceutical excipients. Said pharmaceutical preparations comprise the active agents in an amount ranging from 0.1 % to 90%.

Diacerein may be administered daily in 25, 50, or 100, or 200 mg doses.

Flurbiprofen may be administered daily in 50, 100, or 200, or 300 mg doses.

Loxoprofen may be administered daily in 1, 30, 60, 90 or 120 or 150 mg doses.

Zaltoprofen may be administered daily in 1, 80, 100, 160, 240 or 320 mg doses.

Ibuprofen may be administered daily in 50, 100 or 200, 400 or 600 mg doses.

Ketoprofen or dexketoprofen may be administered daily in 1, 25, 50, 100 or 150, 200 or 300 mg doses.

Naproxen may be administered daily in 200, 250 or 500 or 750 mg doses.

Thus, the present invention provides a synergistic composition, which achieves the objects referred to above and has therapeutic effect on pain, inflammation, fewer, and osteoarthritis.

The pharmaceutical compositions according to the present invention may also comprise one or more pharmaceutically acceptable excipient(s). Such pharmaceutically acceptable excipients include, but are not limited to fillers, glidants, lubricants, disintegrants, surface active agents etc. and the mixtures thereof.

The present invention is for use in mammalians and particularly in humans for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

In this context, the term composition may both correspond to a composition, and to a combined meaning of the composition and the package or blister in which the composition is stored.

It is further possible to use the following additional excipients in the composition.

Diluents, e.g. at least one or a mixture of lactose, microcrystalline cellulose, starch, mannitol, calcium phosphate anhydrate, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch.

Binders, e.g. at least one or a mixture of polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polyethylene oxide, gelatin, starch, xanthan gum, guar gum, alginate, carrageen, pectin, carbomer, cellulose acetate phthalate, hydroxypropyl starch, hydroxyethyl methyl cellulose, polaxomer, polyethylene glycol (PEG).

Lubricants, e.g. at least one or a mixture of sodium stearyl fumarate, polyethylene glycol, stearic acid, metal stearates, boric acid, sodium chloride benzoate and acetate, sodium or magnesium lauryl sulfate, etc..

Preservatives, e.g. at least one or a mixture of methylparaben and propylparaben and salts thereof (e.g. sodium or potassium salts), sodium benzoate, citric acid, benzoic acid, butylated hydroxytoluene and butylated hydroxyanisole, etc..

Surface active agents, e.g. at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate, etc..

The present invention is hereby disclosed by referring to exemplary embodiments hereinabove. Whilst these exemplary embodiments does not restrict the object of the present invention, it must be assessed under the light of the foregoing detailed description.

## Claims

1. A composition for use in the treatment of pain, inflammation, fever, and osteoarthritis, comprising diacerein or a pharmaceutically acceptable salt of diacerein and at least one agent selected from the group of non-steroidal anti-inflammatory agents consisting of flurbiprofen, loxoprofen, zaltoprofen, ibuprofen, naproxen, ketoprofen, dexketoprofen, fenoprofen, benoxaprofen, suprofen, ibuproxam, alminoprofen, dexibuprofen, indoprofen.

2. The composition according to Claim 1, wherein said non-steroidal anti-inflammatory agent is preferably flurbiprofen.

3. The pharmaceutical composition according to any of the preceding claims, wherein said non-steroidal anti-inflammatory agent is preferably loxoprofen.

4. The pharmaceutical composition according to any of the preceding claims, wherein said non-steroidal anti-inflammatory agent is preferably zaltoprofen.

5. The pharmaceutical composition according to any of the preceding claims, wherein said non-steroidal anti-inflammatory agent is preferably ibuprofen.

6. The pharmaceutical composition according to any of the preceding claims, wherein said non-steroidal anti-inflammatory agent is preferably ketoprofen or dexketoprofen.

7. The pharmaceutical composition according to any of the preceding claims, wherein said non-steroidal anti-inflammatory agent is preferably naproxen.

8. The pharmaceutical composition according to any of the preceding claims, wherein the amount of diacerein is 50 - 300 mg/day.

9. The pharmaceutical composition according to any of the preceding claims, wherein the amount of flurbiprofen is 50 - 500 mg/day.

10. The pharmaceutical composition according to any of the preceding claims, wherein the amount of loxoprofen is 50 - 400 mg/day.

11. The pharmaceutical composition according to any of the preceding claims, wherein the amount of zaltoprofen is 50 - 500 mg/day.

12. The pharmaceutical composition according to any of the preceding claims, wherein the amount of ibuprofen is 50 - 1000 mg/day.

13. The pharmaceutical composition according to any of the preceding claims, wherein the amount of ketoprofen or dexketoprofen is 25 - 400 mg/day.

14. The pharmaceutical composition according to any of the preceding claims, wherein the amount of naproxen is 200 - 800 mg/day.

15. The pharmaceutical composition according to any of the preceding claims, wherein the proportion range of diacerein to the non-steroidal anti-inflammatory agents is 0.01 -4.

16. The pharmaceutical composition according to any of the preceding claims, further comprising one selected from nimesulide, s-etodolac, glucosamine, methylsulfonylmethane and chondroitin.

17. The pharmaceutical composition according to any of the preceding claims, wherein said composition is a fixed dose or a kit.

18. The pharmaceutical composition according to any of the preceding claims, wherein said composition is in the form of an oral solid or liquid, or a topical liquid, or a gel.

19. The pharmaceutical composition according to any of the preceding claims, wherein said oral solid form is a tablet or a capsule.

20. The pharmaceutical composition according to any of the preceding claims, further comprising at least one or more excipient(s).

21. The pharmaceutical composition according to any of the preceding claims, wherein said excipient(s) comprise(s) one or more selected from the group consisting of diluents, binders, disintegrants, glidants, lubricants, solvents, pH regulators, gelling agents, and plasticizers.

22. The pharmaceutical composition according to any of the preceding claims, wherein each active agent in said composition is used in sequence, separately or in a fixed combination, either simultaneously or sequentially in any order.

23. The pharmaceutical composition according to any of the preceding claims, wherein said composition comprising diacerein or a pharmaceutically acceptable salt of diacerein and at least one agent selected from the group of non-steroidal anti-inflammatory agents consisting of flurbiprofen, ibuprofen, naproxen, ketoprofen, dexketoprofen, fenoprofen, benoxaprofen, suprofen, ibuproxam, alminoprofen, dexibuprofen, indoprofen is used for manufacturing a drug for the prevention or treatment of pain, arthraigia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

24. The pharmaceutical composition according to any of the preceding claims for use in mammalians and particularly in humans for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

## Patentansprüche

1. Zusammensetzung zur Verwendung bei der Behandlung von Schmerzen, Entzündung, Fieber und Osteoarthritis, umfassend Diacerein oder ein pharmazeutisch akzeptables Salz von Diacerein und mindestens ein Mittel aus der Gruppe nichtsteroidaler entzündungshemmender Mittel, bestehend aus Flurbiprofen, Loxoprofen, Zaltoprofen, Ibuprofen, Naproxen, Ketoprofen, Dexketoprofen, Fenoprofen, Benoxaprofen, Suprofen, Ibuproxam, Alminoprofen, Dexibuprofen, Indoprofen.

2. Zusammensetzung nach Anspruch 1, wobei das nichtsteroidale entzündungshemmende Mittel vorzugsweise Flurbiprofen ist.

3. Pharmazeutische Zusammensetzung nach einem der vorhergehenden Ansprüche, wobei das nichtsteroidale entzündungshemmende Mittel vorzugsweise Loxoprofen ist.

4. Pharmazeutische Zusammensetzung nach einem der vorhergehenden Ansprüche, wobei das nichtsteroidale entzündungshemmende Mittel vorzugsweise Zaltoprofen ist.

5. Pharmazeutische Zusammensetzung nach einem der vorhergehenden Ansprüche, wobei das nichtsteroidale entzündungshemmende Mittel vorzugsweise Ibuprofen ist.

6. Pharmazeutische Zusammensetzung nach einem der vorhergehenden Ansprüche, wobei das nichtsteroidale entzündungshemmende Mittel vorzugsweise Ketoprofen oder Dexketoprofen ist.

7. Pharmazeutische Zusammensetzung nach einem der vorhergehenden Ansprüche, wobei das nichtsteroidale entzündungshemmende Mittel vorzugsweise Naproxen ist.

8. Pharmazeutische Zusammensetzung nach einem der vorhergehenden Ansprüche, wobei die Menge an Diacerein 50 - 300 mg/Tag beträgt.

9. Pharmazeutische Zusammensetzung nach einem der vorhergehenden Ansprüche, wobei die Menge an Flurbiprofen 50 - 500 mg/Tag beträgt.

10. Pharmazeutische Zusammensetzung nach einem der vorhergehenden Ansprüche, wobei die Menge an Loxoprofen 50 - 400 mg/Tag beträgt.

11. Pharmazeutische Zusammensetzung nach einem der vorhergehenden Ansprüche, wobei die Menge an Zaltoprofen 50 - 500 mg/Tag beträgt.

12. Pharmazeutische Zusammensetzung nach einem der vorhergehenden Ansprüche, wobei die Menge an Ibuprofen 50 - 1000 mg/Tag beträgt.

13. Pharmazeutische Zusammensetzung nach einem der vorhergehenden Ansprüche, wobei die Menge an Ketoprofen oder Dexketoprofen 25 - 400 mg/Tag beträgt.

14. Pharmazeutische Zusammensetzung nach einem der vorhergehenden Ansprüche, wobei die Menge an Naproxen 200 - 800 mg/Tag beträgt.

15. Pharmazeutische Zusammensetzung nach einem der vorhergehenden Ansprüche, wobei das Verhältnis von Diacerein zu den nichtsteroidalen entzündungshemmenden Mitteln im Bereich von 0,01 bis 4 liegt.

16. Pharmazeutische Zusammensetzung nach einem der vorhergehenden Ansprüche, ferner umfassend eine, die ausgewählt ist aus Nimesulid, s-Etodolac, Glucosamin, Methylsulfonylmethan und Chondroitin.

17. Pharmazeutische Zusammensetzung nach einem der vorhergehenden Ansprüche, wobei die Zusammensetzung eine feste Dosis oder ein Set ist.

18. Pharmazeutische Zusammensetzung nach einem der vorhergehenden Ansprüche, wobei die Zusammensetzung in Form eines Feststoffs oder einer Flüssigkeit zur oralen Anwendung oder einer Flüssigkeit zur lokalen Anwendung oder eines Gels vorliegt.

19. Pharmazeutische Zusammensetzung nach einem der vorhergehenden Ansprüche, wobei die feste Form zur oralen Anwendung eine Tablette oder eine Kapsel ist.

20. Pharmazeutische Zusammensetzung nach einem der vorhergehenden Ansprüche, ferner umfassend mindestens einen oder mehrere Hilfsstoff(e).

21. Pharmazeutische Zusammensetzung nach einem der vorhergehenden Ansprüche, wobei der bzw. die Hilfsstoff(e) einen oder mehrere umfassen, die aus der aus Verdünnungsmitteln, Bindemitteln, Sprengmitteln, Gleitmitteln, Schmiermitteln, Lösungsmitteln, pH-Regulatoren, Geliermitteln und Weichmachern bestehenden Gruppe ausgewählt sind.

22. Pharmazeutische Zusammensetzung nach einem der vorhergehenden Ansprüche, wobei jeder Wirkstoff in der Zusammensetzung nacheinander, getrennt oder in einer festen Kombination, entweder gleichzeitig oder sequentiell in beliebiger Reihenfolge verwendet wird.

23. Pharmazeutische Zusammensetzung nach einem der vorhergehenden Ansprüche, wobei die Zusammensetzung umfassend Diacerein oder ein pharmazeutisch akzeptables Salz von Diacerein und mindestens ein Mittel aus der Gruppe nichtsteroidaler entzündungshemmender Mittel, bestehend aus Flurbiprofen, Ibuprofen, Naproxen, Ketoprofen, Dexketoprofen, Fenoprofen, Benoxaprofen, Suprofen, Ibuproxam, Alminoprofen, Dexibuprofen, Indoprofen zur Herstellung eines Arzneimittels zur Vorbeugung oder Behandlung von Schmerzen, Arthraglie, Zahnschmerzen, Myalgie, Miosishemmung, ankylosierender Spondylitis, Osteoarthritis, rheumatoider Arthritis und anderen Erkrankungen des Muskel- und Skelettsystems sowie der Gelenke, Weichteilverletzungen wie zum Beispiel Verstauchungen und Zerrungen, postoperativen Schmerzen, Menstruationsschmerzen und -beschwerden, Migräne und Halsschmerzen verwendet wird.

24. Pharmazeutische Zusammensetzung nach einem der vorhergehenden Ansprüche zur Verwendung bei Säugetieren und insbesondere bei Menschen zur Vorbeugung oder Behandlung von Schmerzen, Arthralgie, Zahnschmerzen, Myalgie, Miosishemmung, ankylosierender Spondylitis, Osteoarthritis, rheumatoider Arthritis und anderen Erkrankungen des Muskel- und Skelettsystems sowie der Gelenke, Weichteilverletzungen wie zum Beispiel Verstauchungen und Zerrungen, postoperativen Schmerzen, Menstruationsschmerzen und -beschwerden, Migräne und Halsschmerzen.

## Revendications

1. Composition destinée à être utilisée pour le traitement de la douleur, de l'inflammation, de la fièvre, et de l'arthrose, comprenant de la diacéréine ou un sel pharmaceutiquement acceptable de la diacéréine et au moins un agent sélectionné à partir du groupe d'agents anti-inflammatoires non stéroïdiens constitué du flurbiprofène, du loxoprofène, du zaltoprofène, de l'ibuprofène, du naproxène, du kétoprofène, du dexkétoprofène, du fénoprofène, du bénoxaprofène, du suprofène, de l'ibuproxam, de l'alminoprofène, du dexibuprofène, de l'indoprofène.

2. Composition selon la revendication 1, dans laquelle ledit agent anti-inflammatoire non stéroïdien est de préférence du flurbiprofène.

3. Composition pharmaceutique selon l'une quelconque des revendications précédentes, dans laquelle ledit agent anti-inflammatoire non stéroïdien est de préférence du loxoprofène.

4. Composition pharmaceutique selon l'une quelconque des revendications précédentes, dans laquelle ledit agent anti-inflammatoire non stéroïdien est de préférence du zaltoprofène.

5. Composition pharmaceutique selon l'une quelconque des revendications précédentes, dans laquelle ledit agent anti-inflammatoire non stéroïdien est de préférence de l'ibuprofène.

6. Composition pharmaceutique selon l'une quelconque des revendications précédentes, dans laquelle ledit agent anti-inflammatoire non stéroïdien est de préférence du kétoprofène ou du dexkétoprofène.

7. Composition pharmaceutique selon l'une quelconque des revendications précédentes, dans laquelle ledit agent anti-inflammatoire non stéroïdien est de préférence du naproxène.

8. Composition pharmaceutique selon l'une quelconque des revendications précédentes, dans laquelle la dose de diacéréine est de 50 à 300 mg/jour.

9. Composition pharmaceutique selon l'une quelconque des revendications précédentes, dans laquelle la dose de flurbiprofène est de 50 à 500 mg/jour.

10. Composition pharmaceutique selon l'une quelconque des revendications précédentes, dans laquelle la dose de loxoprofène est de 50 à 400 mg/jour.

11. Composition pharmaceutique selon l'une quelconque des revendications précédentes, dans laquelle la dose de zaltoprofène est de 50 à 500 mg/jour.

12. Composition pharmaceutique selon l'une quelconque des revendications précédentes, dans laquelle la dose d'ibuprofène est de 50 à 1000 mg/jour.

13. Composition pharmaceutique selon l'une quelconque des revendications précédentes, dans laquelle la dose de kétoprofène ou de dexkétoprofène est de 25 à 400 mg/jour.

14. Composition pharmaceutique selon l'une quelconque des revendications précédentes, dans laquelle la dose de naproxène est de 200 à 800 mg/jour.

15. Composition pharmaceutique selon l'une quelconque des revendications précédentes, dans laquelle la proportion entre la diacéréine et les agents anti-inflammatoires non stéroïdiens est de 0,01 à 4.

16. Composition pharmaceutique selon l'une quelconque des revendications précédentes, comprenant en outre un agent sélectionné parmi le nimésulide, le s-etodolac, la glucosamine, le méthylsulfonylméthane et la chondroïtine.

17. Composition pharmaceutique selon l'une quelconque des revendications précédentes, dans laquelle ladite composition est une dose fixe ou un kit.

18. Composition pharmaceutique selon l'une quelconque des revendications précédentes, dans laquelle ladite composition se présente sous la forme d'un solide ou d'un liquide pour administration orale, ou d'un liquide topique, ou d'un gel.

19. Composition pharmaceutique selon l'une quelconque des revendications précédentes, dans laquelle ladite forme solide pour administration orale est un comprimé ou une capsule.

20. Composition pharmaceutique selon l'une quelconque des revendications précédentes, comprenant en outre au moins un ou plusieurs excipient(s).

21. Composition pharmaceutique selon l'une quelconque des revendications précédentes, dans laquelle ledit ou lesdits excipient(s) comprennent un ou plusieurs sélectionnés dans le groupe constitué de diluants, liants, agents désintégrants, agents de glissement, lubrifiants, solvants, régulateurs de pH, agents gélifiants, et plastifiants.

22. Composition pharmaceutique selon l'une quelconque des revendications précédentes, dans laquelle chaque agent actif dans ladite composition est utilisé en séquence, séparément ou dans une combinaison fixe, soit simultanément ou séquentiellement dans n'importe quel ordre.

23. Composition pharmaceutique selon l'une quelconque des revendications précédentes, dans laquelle ladite composition comprend de la diacéréine ou un sel pharmaceutiquement acceptable de la diacéréine et au moins un agent sélectionné dans le groupe d'agents anti-inflammatoires non stéroïdiens constitué du flurbiprofène, de l'ibuprofène, du naproxène, du kétoprofène, du dexkétoprofène, du fénoprofène, du bénoxaprofène, du suprofène, de l'ibuproxam, du alminoprofène, du dexibuprofène, de l'indoprofène est utilisé dans la fabrication d'un médicament destiné à la prévention ou au traitement de la douleur, l'arthralgie, le mal de dents, la myalgie, l'inhibition du myosis, la spondylarthrite ankylosante, l'arthrose, la polyarthrite rhumatoïde et autres troubles du système muscolosquelettique et des articulations, les lésions des tissus mous telles que foulures et entorses, les douleurs postopératoires, les règles douloureuses et sévères, la migraine, et le mal de gorge.

24. Composition pharmaceutique selon l'une quelconque des revendications précédentes pour une utilisation chez les mammifères et en particulier chez les humains, destinée à la prévention ou au traitement de la douleur, l'arthralgie, le mal de dents, la myalgie, l'inhibition du myosis, la spondylarthrite ankylosante, l'arthrose, la polyarthrite rhumatoïde and autres troubles du système muscolosquelettique et des articulations, les lésions des tissus mous telles que foulures et entorses, les douleurs postopératoires, les règles douloureuses et sévères, la migraine, et le mal de gorge.
